# EUROPEAN PATENT SPECIFICATION

(11) **EP 1 097 223 B1**
(45) Date of publication and mention of the grant of the patent: **21.02.2007**
(21) Application number: 99933797.5
(22) Date of filing: 10.07.1999
(51) Int. Cl.: C12N 15/54, C12N 15/82, A01H 5/00, A01H 5/10

(54) **EXPRESSION OF HERBICIDE TOLERANCE GENES IN PLANT PLASTIDS**
EXPRESSION VON HERBIZIDTOLERANZGENEN IN PFLANZENPLASTIDEN
EXPRESSION GENIQUE DE TOLERANCE AUX HERBICIDES DANS LES PLASTES DE VEGETAUX

(30) Priority: 10.07.1998 US 113257
(43) Date of publication of application: 09.05.2001
(73) Proprietor: Calgene LLC, Davis, CA 95616 (US)
(72) Inventor: HAJDUKIEWICZ, Peter, Chesterfield, MO 60317 (US); MCBRIDE, Kevin, E., Davis, CA 95616 (US); NEHRA, Narender, Chesterfield, MO 63017 (US); SCHAAF, David, J., Davis, CA 95616 (US); STALKER, David, M., Woodland, CA 95695 (US); STAUB, Jeffrey, M., Wildwood, Missouri 63011 (US); YE, Guangning, Ellisville, MO 63011 (US)
(74) Representative: Helbing, Jörg
(86) International application number: PCT/US1999/015472
(87) International publication number: WO 2000/003022

(56) References cited:
- EP-A- 0 293 358
- EP-A- 0 337 899
- WO-A-92/04449
- WO-A-95/16783
- WO-A-95/24493
- WO-A-97/04103
- WO-A-97/32011
- WO-A-98/11235
- WO-A-98/20144
- WO-A-99/05265
- US-A- 5 589 612
- US-A- 5 633 437
- US-A- 5 693 507
- DANIELL ET AL: "Containment of herbicide resistance through genetic engineering of the chloroplast genome" NATURE BIOTECHNOLOGY,US,NATURE PUBLISHING, vol. 16, April 1998 (1998-04), page 345-348 XP002090409 ISSN: 1087-0156
- DATTA ET AL: "Transformation of the tobacco chloroplast genome with the aroA gene to confer glyphosate tolerance" PLANT PHYSIOLOGY,US,AMERICAN SOCIETY OF PLANT PHYSIOLOGISTS, ROCKVILLE, MD, vol. 111, no. 2, 1996, page 168 XP002090406 ISSN: 0032-0889
- MCBRIDE ET AL: "Amplification of a chimeric Bacillus gene in chloroplasts leads to an extraordinary level of an insecticial protein in tobacco" BIO/TECHNOLOGY,US,NATURE PUBLISHING CO. NEW YORK, vol. 13, 1995, page 362-365 XP002090404 ISSN: 0733-222X
- ZHOU H ET AL: "GLYPHOSATE-TOLERANT CP4 AND GOX GENES AS A SELECTABLE MARKER IN WHEAT TRANSFORMATION" PLANT CELL REPORTS,DE,SPRINGER VERLAG, vol. 15, no. 3/04, 1 December 1995 (1995-12-01), pages 159-163, XP000578832 ISSN: 0721-7714
- MISAWA N ET AL: "FUNCTIONAL EXPRESSION OF THE ERWINIA UREDOVORA CAROTENOID BIOSYNTHESIS GENE CRTL IN TRANSGENIC PLANTS SHOWING AN INCREASE OF BETA-CAROTENE BIOSYNTHESIS ACTIVITY AND RESISTANCE TO THE BLEACHING HERBICIDE NORFLURAZON" PLANT JOURNAL,GB,BLACKWELL SCIENTIFIC PUBLICATIONS, OXFORD, vol. 4, no. 5, 1 January 1993 (1993-01-01), pages 833-840, XP002017202 ISSN: 0960-7412
- SVAB Z.; MALIGA P. PROC. NATL. ACAD. SCI vol. 90, 1993, pages 913 - 917, XP002106110
- SHINOZAKI ET AL EMBO J. vol. 5, 1986, pages 2043 - 2049, XP001014134
- STAUB J.; MALIGA P. EMBO J. vol. 12, no. 2, 1993, pages 601 - 606, XP001182918

### SEQUENCE LISTING

<110> Calgene LLC
<120> Expression of Hericide Tolerance Genes in Plant Plastids
<130> 15336/00/WO
<140> PCT/US 99/15472
   <141> 1999-07-09
<150> 09/113,257
   <151> 1998-07-10
<160> 3
<170> FastSEQ for Windows Version 4.0
<210> 1
   <211> 44
   <212> DNA
   <213> Artificial Sequence
<220>
   <223> Synthetic Oligonucleotide
<400> 1
   aattgtagaa ataattttgt ttaactttaa gaaggagata tacc 44
<210> 2
   <211> 44
   <212> DNA
   <213> Artificial Sequence
<220>
   <223> Synthetic Oligonucleotide
<400> 2
   catgggtata tctccttctt aaagttaaac aaaattattt ctac 44
<210> 3
   <211> 58
   <212> PRT
   <213> Human
<400> 3

## Description

### INTRODUCTION

### Technical Field

This invention relates to the application of genetic engineering techniques to plants. Specifically, the invention relates to compositions and methods for enhancing expression of proteins in plant plastids.

### Background

The plastids of higher plants are an attractive target for genetic engineering. Plant plastids (chloroplasts, amyloplasts, elaioplasts, etioplasts, chromoplasts, etc.) are the major biosynthetic centers that, in addition to photosynthesis, are responsible for production of industrially important compounds such as amino acids, complex carbohydrates, fatty acids, and pigments. Plastids are derived from a common precursor known as a proplastid and thus the plastids present in a given plant species all have the same genetic content. Plant cells contain 500-10,000 copies of a small 120-160 kilobase circular genome, each molecule of which has a large (approximately 25kb) inverted repeat. Thus, it is possible to engineer plant cells to contain up to 20,000 copies of a particular gene of interest which potentially can result in very high levels of foreign gene expression. In addition, plastids of most plants are maternally inherited. Consequently, unlike heterologous genes expressed in the nucleus, heterologous genes expressed in plastids are not pollen disseminated, therefore, a trait introduced into a plant plastid will not be transmitted to wild-type relatives.

There remains a need for improved regulatory elements for expression of genes in a plant plastid. To date, the expression signals used routinely for plastid transgene expression derive from endogenous plastid genes. The plastid expression signals are typically derived from promoter regions of highly expressed plastid genes such as the promoter regions from the 16S ribosomal RNA operon (P*rrn*), psbA gene (PpsbA) or the rbcL gene (PrbcL). The psbA and rbcL genes are highly transcribed, but their translation is controlled by tissue-specific and light-regulated factors which limits their usefulness. In the case of P*rrn*, a synthetic ribosome binding site (RBS) patterned after the plastid rbcL gene leader has been typically used to direct translation. However, this P*rrn*/RBS is translated inefficiently due to poor ribosome binding.

A totally heterologous expression system has been used to express plastid genes (USPN 5,576,198). This system is a two component system. The first component is a plastid transgene driven by a T7 bacteriophage gene 10 promoter/leader sequence. The second component is a nuclear gene encoding the T7 Polymerase that is targeted to the plastid compartment. The limitation of this system is the need to create nuclear transformed lines that express the T7 Polymerase in preferred ways.

Plastids of higher plants present an attractive target for genetic engineering. As mentioned above, plastids of higher plants are maternally inherited. This offers an advantage for genetic engineering of plants for tolerance or resistance to natural or chemical conditions, such as herbicide tolerance, as these traits will not be transmitted to wild-type relatives. In addition, the high level of foreign gene expression is attractive for engineered traits such as the production of pharmaceutically important proteins.

Expression of nucleic acid sequences encoding for enzymes providing for herbicide tolerance as well as pharmaceutical proteins from plant plastid genome offers an attractive alternative to expression from the plant nuclear genome.

### SUMMARY OF THE INVENTION

The present invention provides nucleic acid sequences useful in enhancing expression of a particular EPSPS genes in plant plastids. Furthermore, plastid expression constructs are provided which are useful for genetic engineering of plant cells and which provide for enhanced expression of the EPSP synthase protein in plant cell plastids. The transformed plastids should be metabolically active plastids, and are preferably maintained at a high copy number in the plant tissue of interest, most preferably the chloroplasts found in green plant tissues, such as leaves or cotyledons.

The plastid expression constructs for use in this invention include a plastid promoter sequence of the 16S ribosomal RNA operon (Prrn) capable of providing for enhanced expression of the DNA sequence encoding the EPSPS protein, and a transcription termination region capable of terminating transcription in a plant plastid.

The plastid promoter region of the present invention is linked to a ribosome binding site of the T7 bacteriophage polymerase gene 10 leader which provides for enhanced translation of mRNA transcripts in a plant plastid.

The plastid expression construct of this invention is preferably linked to a construct having a DNA sequence encoding a selectable marker which can be expressed in a plant plastid. Expression of the selectable marker allows the identification of plant cells comprising a plastid expressing the marker.

In a preferred embodiment, vectors for transfer of the construct into a plant cell include means for inserting the expression and selection constructs into the plastid genome. This preferably comprises regions of homology to the target plastid genome which flank the constructs.

The constructs of the present invention as defined comprises a promoter sequence aboved linked to a ribosome binding site as defined above capable of enhancing the translation of mRNA transcripts in the plant plastid.

Of particular interest in the present invention is the high level of expression of particular EPSPS nucleic acid sequences in plant plastids.

The constructs of the present invention preferably comprises a DNA sequence encoding for a 5-Enolpyruvylshikimate-3-phosphate synthase (USPN 5,633,435).

Plant cell plastids containing the constructs are also contemplated in the invention, as are plants, plant seeds, plant cells or progeny thereof containing plastids comprising the construct.

The present invention also includes methods for enhanced expression of DNA sequences in plant plastids.

The invention also includes a method for the enhanced expression of an enzyme conferring herbicide tolerance in a plant cell, by expressing the *Agrobacterium tumefaciens* sp strain CP4 EPSPS in plastids of the plant cell.

Thus, the present invention relates to a chimeric gene containing a particularly herbicide tolerance coding sequence, a plant plastid expression vector containing a prrn promoter operably linked to a T7 Bacteriophage Polymerase gene 10 ribosome binding site capable of enhanced expression in a plant plastid operably linked to a herbicide tolerance or pharmaceutical coding gene, a plant transformation vector having inserted therein a particular herbicide tolerance gene expressed from a plastid promoter linked to a T7 Bacteriophage Polymerase gene 10 ribosome binding site, plant cells transformed using such vectors and plants regenerated therefrom which exhibit a substantial degree of expression of nucleic acid sequences and proteins and methods for producing such plants and such plants.

### BRIEF DESCRIPTION OF THE DRAWINGS

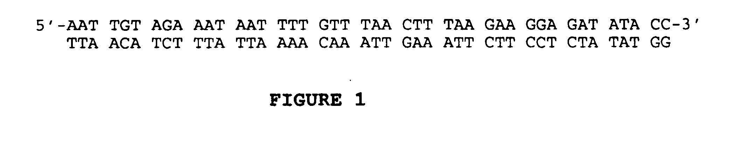

Figure 1 shows the nucleotide sequence of the G10L ribosome binding site.

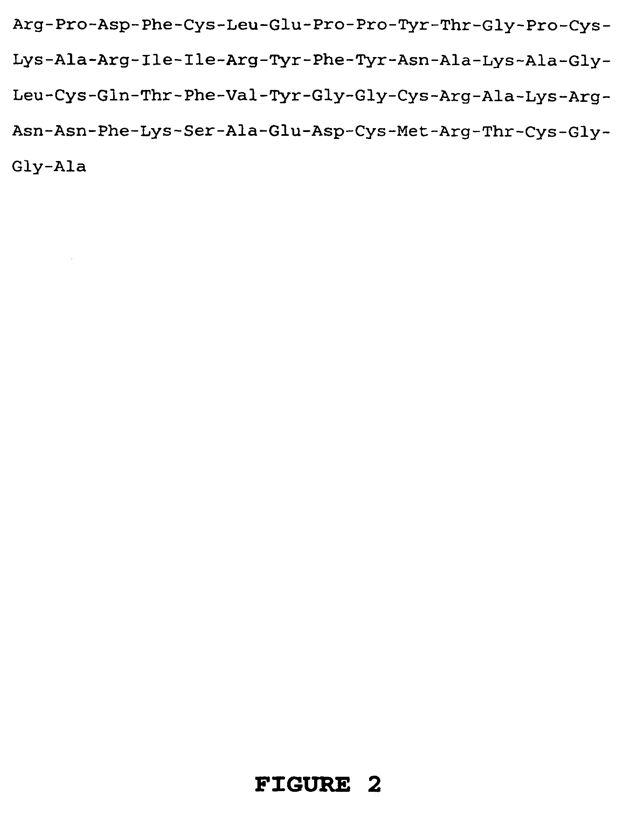

Figure 2 provides an amino acid sequence encoding for aprotinin.

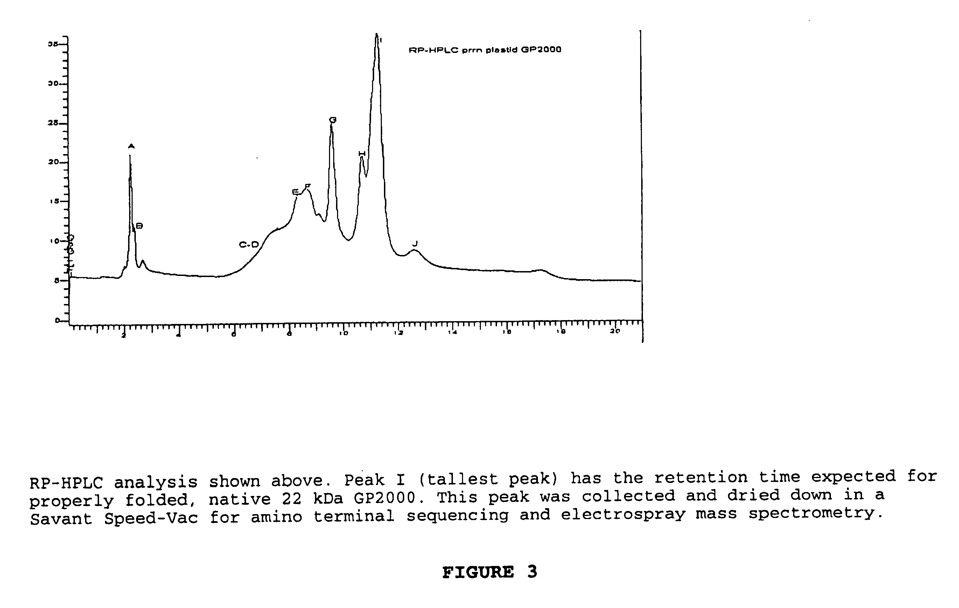

Figure 3 provides the results of RP-HPLC analysis for characterization ofhGH protein expressed in the plastid. Peak I (tallest peak) indicates the expected retention time for properly folded, native 22 kDa GP2000.

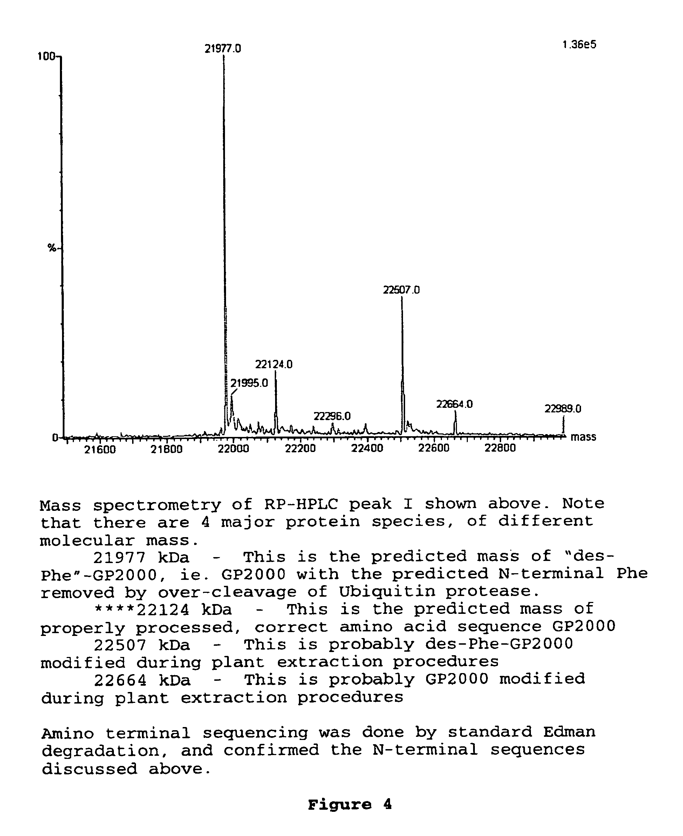

Figure 4 provides an electrospray ionization mass spectrometry (MS) analysis using a Micromass Q-Tof electrospray time-of-flight mass spectrometer. In particular, a series of ions corresponding to the specie(s) present in the sample with varying numbers of protons attached is provided. The axes of the spectrum are intensity versus mass-to-charge ratio of the specie(s) present.

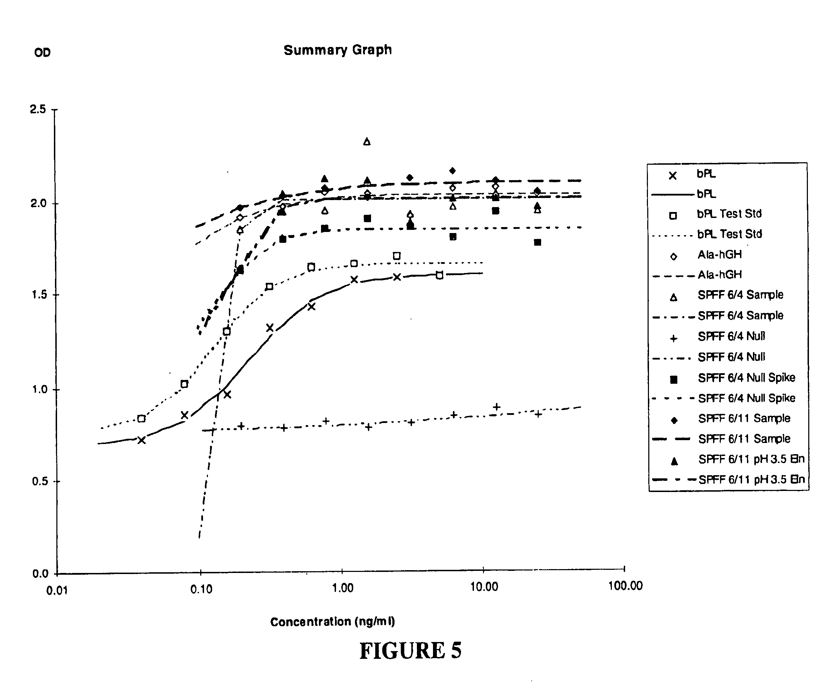

Figure 5 provides a graphic representation of the bioactivity of hGH expressed from a plant plastid. The samples represented on the graph are bovine prolactin (bPL), hGH expressed from *E. coli* (Ala-hGH), and a null transgenic spiked with bovine prolactin (SPFF Null Spike) as positive controls, a null transgenic (SPFF Null) as a negative control, and transgenic samples from a sepharose column (SPFF Sample, SPFF Sample) and a transgenic sample eluted from the sepharose column at pH3.5 (SPFF pH3.5 Eln).

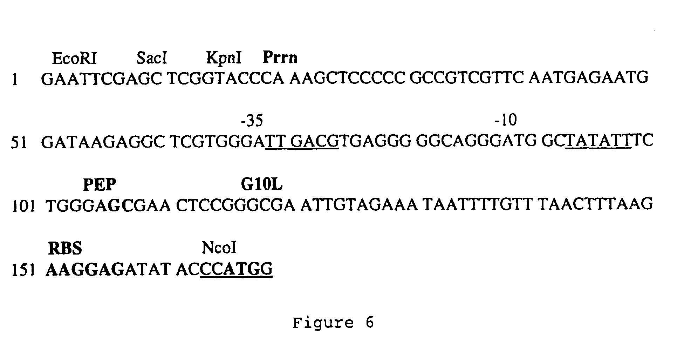

Figure 6 provides the nucleic acid sequence for the Prrn/G10L promoter/RBS hybrid. The Prrn promoter contains the consensus plastid -35 and -10 promoter elements (underlined) and the transcription start sites (GC in bold) for the Plastid-Encoded RNA Polymerase (PEP). The gene 10 leader (G10L) contains a perfect plastid ribosome binding site (RBS, nucleotides in bold).

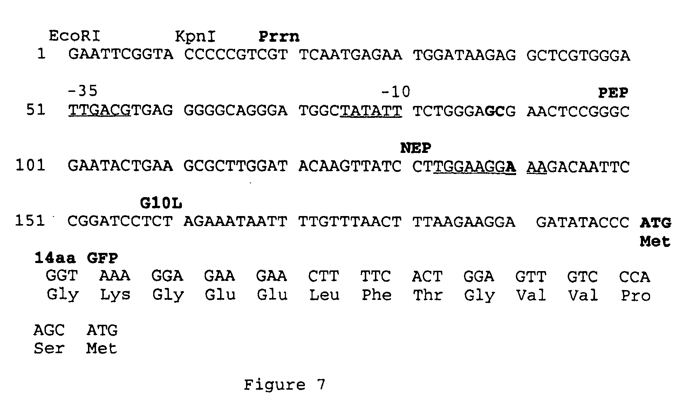

Figure 7 provides the nucleic acid sequence for the Prrn/NEP/G10L::14aaGFP fusion. The NEP promoter region is underlined (A in bold is transcription start site). The NEP promoter region used extends beyond the consensus sequence both upstream and downstream of the promoter. The initial ATG, the initiator methionine is not counted in the 14 amino acids of GFP.

### DETAILED DESCRIPTION OF THE INVENTION

In accordance with the subject invention, plastid expression constructs are provided which generally comprise a promoter functional in a plant plastid, a ribosome binding site derived from the T7 Bacteriophage Polymerase gene 10 leader, a DNA sequence encoding a particular EPSPS, and a transcription termination region capable of terminating transcription in a plant plastid. These elements are provided as operably joined components in the 5' to 3' direction of transcription.

Furthermore, the constructs of the present invention may also include a nucleic acid sequence encoding a peptide capable of targeting said DNA sequence encoding a protein to the thylakoid lumen within the chloroplast.

Of particular interest in the present invention is the use of the plastid expression constructs to direct the high level transcription and translation (expression) of nucleic acid sequences. Such plastid expression constructs find use in directing the high level expression of DNA sequences encoding for particular EPSPS enzymes involved in herbicide tolerance.

Of more particular interest in the present invention is the use of the plastid expression constructs to direct the high level translation of transcribed messenger RNA.

DNA sequence and biochemical data reveal a similarity of the plastid organelle's transcriptional and translational machineries and initiation signals to those found in prokaryotic systems. In fact, plastid derived promoter sequences have been reported to direct expression of reporter genes in prokaryotic cells. In addition, plastid genes are often organized into polycistronic operons as they are in prokaryotes.

Despite the apparent similarities between plastids and prokaryotes, there exist fundamental differences in the methods used to control gene expression in plastids and prokaryotes. As opposed to the transcriptional control mechanisms typically observed in prokaryotes, plastid gene expression is controlled predominantly at the level of translation and mRNA stability by trans-acting nuclear encoded proteins.

Translation is a multi-stage process which first involves the binding of messenger RNA (mRNA) to ribosomes. Beginning at the translation start codon, the mRNA codons are read sequentially as the ribosomes move along the mRNA molecule. The specified amino acids are then sequentially added to the growing polypeptide chain to yield the protein or polypeptide encoded in the mRNA.

As mentioned, the first step in the translation process is the binding of the mRNA molecule to the ribosome. The nature of this interaction (i.e. binding) has been only partially elucidated. Analysis of RNase-resistant oligonucleotides isolated from bacterial translation initiation complexes indicate that a RNA fragment approximately 30 to 40 nucleotides in length comprises the initial ribosome binding site (RBS). Thus, a RBS is hereinafter understood to comprise a sequence of mRNA surrounding the translation start codon which is responsible for the binding of the ribosome and for initiation of translation.

Recently, ribosome binding sites have been identified capable of directing translation in a prokaryotes. For example, a ribosome binding site derived from the T7 bacteriophage gene 10 leader. G10L (USPN 5,232,840), has been identified which enhances expression of nucleic acid sequences in prokaryotes.

Herbicides such as N-phosphonomethylglycine, halogenated hydroxybenzonitriles, and norflurazon have been the subject of a large amount of investigation.

N-phosphonomethylglycine, commonly referred to as glyphosate, inhibits the shikimic acid pathway which leads to the biosynthesis of aromatic compounds including amino acids, plant hormones and vitamins. Specifically, glyphosate curbs the conversion of phosphoenolpyruvic acid (PEP) and 3-phosphoshikimic acid to 5-enolpyruvyl-3-phosphoshikimic acid by inhibiting the enzyme 5-enolpyruvylshikimate-3-phosphate synthase (hereinafter referred to as EPSP synthase or EPSPS).

Glyphosate tolerant plants have been produced by transformation of various EPSP synthase genes into the nuclear genome of a plant. A gene for EPSP synthase has been cloned from *Agrobacterium tumefaciens* sp strain CP4 (USPN 5,633,435) and confers a high level of glyphosate tolerance in plants. Furthermore, high levels of glyphosate tolerance has been achieved in a number of crop plants by fusing EPSPS to a chloroplast transit peptide (CTP) for targeted expression in plastids. In addition, variants of the wild-type EPSPS enzyme have been isolated which are glyphosate tolerant as a result of alterations in the EPSPS amino acid coding sequence (Kishore and Shah, *Ann. Rev. Biochem.* (1988) 57:627-663; Shulze et al.,*Arch. Microbiol.* (1984) 137:121-123; Kishore et al., *Fed. Proc.* (1986) 45:1506). These variants typically have a higher Kᵢ for glyphosate than the wild-type EPSPS enzyme which confers the glyphosate tolerant phenotype, but these variants are also characterized by a high Kₘ for PEP which makes the enzyme kinetically less efficient (Kishore and Shah, *Ann. Rev. Biochem.* (1988) 57:627-663; Sost et al., *FEBS Lett.* (1984) 173: 238-24 1; Shulze et al.,*Arch. Microbiol*. (1984) 137:121-123; Kishore et al., *Fed. Proc.* (1986) 45:1506; Sost and Amrhein, *Arch. Biochem. Biophys.* (1990) 282: 433-436).

While plants transformed to express nucleic acid sequences encoding for such enzymes from the nuclear genome have found utility in engineering herbicide tolerant plants, it would be increasingly beneficial to obtain herbicide tolerant plants via plastidial expression.

In the examples provided herein, DNA sequences encoding for enzymes involved in herbicide tolerance are used in constructs to direct the expression of the sequences from the plant plastid. DNA sequences encoding for 5-enolpyruvylshikimate-3-phosphate synthase (EPSPS), are used in the expression constructs of the present invention to direct the expression of said herbicide tolerance nucleotide sequences from the plant plastid.

Transplastomic tobacco plants are identified which are homoplasmic for the DNA sequences of interest encoding said herbicide tolerance genes. Homoplasmic plants demonstrate a high level of protein expression from the plastid. Furthermore, homoplasmic plants demonstrate a high level of tolerance for the respective herbicide. For example, as described in more detail in the example below, plants transformed to express EPSPS from the plastid demonstrate a high level of tolerance for the herbicide glyphosate.

In developing the constructs the various fragments comprising the regulatory regions and open reading frame may be subjected to different processing conditions, such as ligation, restriction enzyme digestion, PCR, *in vitro* mutagenesis, linkers and adapters addition, and the like. Thus, nucleotide transitions, transversions, insertions or deletions may be performed on the DNA which is employed in the regulatory regions or the DNA sequences of interest for expression in the plastids. Methods for restriction digests, Klenow blunt end treatments, ligations, and the like are well known to those in the art and are described, for example, by Maniatis *et al.* (in *Molecular cloning: a laboratory manual* (1989) Cold Spring Harbor Laboratory Press, Cold Spring Harbor, NY).

During the preparation of the constructs, the various fragments of DNA will often be cloned in an appropriate cloning vector, which allows for amplification of the DNA, modification of the DNA or manipulation of the DNA by joining or removing sequences, linkers, or the like. Preferably, the vectors will be capable of replication to at least a relatively high copy number in *E. coli.* A number of vectors are readily available for cloning, including such vectors as pBR322, vectors of the pUC series, the M13 series vectors, and pBluescript vectors (Stratagene; La Jolla. CA).

In order to provide a means of selecting the desired plant cells, vectors for plastid transformation typically contain a construct which provides for expression of a selectable marker gene. Marker genes are plant-expressible DNA sequences which express a polypeptide which resists a natural inhibition by, attenuates, or inactivates a selective substance, *i.e.*, antibiotic, herbicide.

Alternatively, a marker gene may provide some other visibly reactive response, *i*.*e*., may cause a distinctive appearance or growth pattern relative to plants or plant cells not expressing the selectable marker gene in the presence of some substance, either as applied directly to the plant or plant cells or as present in the plant or plant cell growth media.

In either case, the plants or plant cells containing such selectable marker genes will have a distinctive phenotype for purposes of identification, *i*.*e*., they will be distinguishable from non-transformed cells. The characteristic phenotype allows the identification of cells, cell groups, tissues, organs, plant parts or whole plants containing the construct.

Detection of the marker phenotype makes possible the selection of cells having a second gene to which the marker gene has been linked. This second gene typically comprises a desirable phenotype which is not readily identifiable in transformed cells, but which is present when the plant cell or derivative thereof is grown to maturity, even under conditions wherein the selectable marker phenotype itself is not apparent.

The use of such a marker for identification of plant cells containing a plastid construct has been described by Svab *et al*. (1993, *supra*). In the examples provided below, a bacterial *aad*A gene is expressed as the marker under the regulatory control of chloroplast 5' promoter and 3' transcription termination regions, specifically the regulatory regions of the *psb*A gene (described in Staub *et al., EMBO J*.(1993) 12(2):601-606). Numerous additional promoter regions may also be used to drive expression of the selectable marker gene, including various plastid promoters and bacterial promoters which have been shown to function in plant plastids.

Expression of the *aad*A gene confers resistance to spectinomycin and streptomycin, and thus allows for the identification of plant cells expressing this marker. The *aad*A gene product allows for continued growth and greening of cells whose chloroplasts comprise the selectable marker gene product. Cells which do not contain the selectable marker gene product are bleached. Selection for the *aad*A gene marker is thus based on identification of plant cells which are not bleached by the presence of streptomycin, or more preferably spectinomycin, in the plant growth medium.

A number of markers have been developed for use with plant cells, such as resistance to chloramphenicol, the aminoglycoside G418 or, hygromycin. Other genes which encode a product involved in chloroplast metabolism may also be used as selectable markers. For example, genes which provide resistance to plant herbicides such as glyphosate, bromoxynil or imidazolinone may find particular use. Such genes have been reported (Stalker *et al., J. Biol. Chem*. (1985) 260:4724-4728 (glyphosate resistant EPSP); Stalker *et al., J. Biol. Chem*. (1985) 263:6310-6314 (bromoxynil resistant nitrilase gene); and Sathasivan *et al., Nucl. Acids Res.* (1990) *18*:2188 (AHAS imidazolinone resistance gene)).

Stable transformation of tobacco plastid genomes by particle bombardment is reported (Svab *et.al*. (1990), *supra*) and Svab *et al.* (1993), *supra*). The methods described therein may be employed to obtain plants homoplasmic for plastid expression constructs.

Generally, bombarded tissue is cultured for approximately 2 days on a cell division-promoting media, after which the plant tissue is transferred to a selective media containing an inhibitory amount of the particular selective agent, as well as the particular hormones and other substances necessary to obtain regeneration for that particular plant species. Shoots are then subcultured on the same selective media to ensure production and selection of homoplasmic shoots.

Transplastomic tobacco plants are analyzed for a pure population of transformed plastid genomes (homoplasmic lines). Homoplasmy is verified using Southern analysis employing nucleic acid probes spanning a region of the transgene and chloroplast genome (i.e. the insertion region). Transplastoimc plants which are heteroplasmic (i.e. contain a mixture of plastid genomes containing and lacking the transgene) are characterized by a hybridization pattern of wild type and transgenic bands. Homoplasmic plants show a hybridization pattern lacking the wild type band.

Alternatively, homoplasmy may be verified using the polymerase chain reaction (PCR). PCR primers are utilized which are targeted to amplify from sequences from the insertion region. For example, a pair of primers may be utilized in a PCR reaction. One primer amplifies from a region in the transgene, while the second primer amplifies from a region proximal to the insertion region towards the insertion region. A second PCR reaction is performed using primers designed to amplify the region of insertion. Transplastomic lines identified as homoplasmic produce the expected size fragment in the first reaction, while they do not produce the predicted size fragment in the second reaction.

Where transformation and regeneration methods have been adapted for a given plant species, either by *Agrobacterium*-mediated transformation, bombardment or some other method, the established techniques may be modified for use in selection and regeneration methods to produce plastid-transformed plants. For example, the methods described herein for tobacco are readily adaptable to other solanaceous species, such as tomato, petunia and potato.

For transformation of soybean, particle bombardment as well as *Agrobacterium-*mediated nuclear transformation and regeneration protocols have been described (Hinchee *et al*. USPN 5,416,011, and Christou *et al*. USPN 5,015,580). The skilled artisan will recognize that protocols described for soybean transformation may be used

In *Brassica,* Agrobacterium-mediated transformation and regeneration protocols generally involve the use of hypocotyl tissue, a non-green tissue which might contain a low plastid content. Thus, for *Brassica,* preferred target tissues would include microspore-derived hypocotyl or cotyledonary tissues (which are green and thus contain numerous plastids) or leaf tissue explants. While the regeneration rates from such tissues may be low, positional effects, such as seen with *Agrobacterium*-mediated transformation, are not expected, thus it would not be necessary to screen numerous successfully transformed plants in order to obtain a desired phenotype.

For cotton, transformation of *Gossypium hirsutum* L. cotyledons by co-cultivation with *Agrobacterium tumefaciens* has been described by Firoozabady *et al., Plant Mol. Bio*. (1987) *10*:105-116 and Umbeck *et al., Bio*/*Technology* (1987) 5:263-266. Again, as for *Brassica*, this tissue may contain insufficient plastid content for chloroplast transformation. Thus, as for *Brassica,* an alternative method for transformation and regeneration of alternative target tissue containing chloroplasts may be desirable, for instance targeting green embryogenic tissue.

Other plant species may be similarly transformed using related techniques. Alternatively, microprojectile bombardment methods, such as described by Klein *et al*. (*Bio*/*Technology 10*:286-291) may also be used to obtain nuclear transformed plants comprising the viral single subunit RNA polymerase expression constructs described herein. Cotton transformation by particle bombardment is reported in WO 92/15675, published September 17, 1992. Suitable plants for the practice of the present invention include, but are not limited to, soybean, cotton, alfalfa, oil seed rape, flax, tomato, sugar beet, sunflower, potato, tobacco, maize, wheat, rice and lettuce.

The vectors for use in plastid transformation preferably include means for providing a stable transfer of the plastid expression construct and selectable marker construct into the plastid genome. This is most conveniently provided by regions of homology to the target plastid genome. The regions of homology flank the construct to be transferred and provide for transfer to the plastid genome by homologous recombination, via a double crossover into the genome. The complete DNA sequence of the plastid genome of tobacco has been reported (Shinozaki *et al., EMBO J.* (1986) *5*:2043-2049). Complete DNA sequences of the plastid genomes from liverwort (Ohyama *et al., Nature* (1986) *322*:572-574) and rice (Hiratsuka *et al., Mol. Gen. Genet.* (1989) *217*:185-194), have also been reported.

Where the regions of homology are present in the inverted repeat regions of the plastid genome (known as IRA and IRB), two copies of the transgene are expected per transformed plastid. Where the regions of homology are present outside the inverted repeat regions of the plastid genome, one copy of the transgene is expected per transformed plastid. The regions of homology within the plastid genome are approximately 1kb in size. Smaller regions of homology may also be used, and as little as 100 bp can provide for homologous recombination into the plastid genome. However, the frequency of recombination and thus the frequency of obtaining plants having transformed plastids decreases with decreasing size of the homology regions.

Examples of constructs having regions of homology the plastid genome are described in Svab *et.al*. (1990 *supra*), Svab *et al*. (1993 *supra*) and Zoubenko *et al.* (*Nuc Acid Res* (1994) 22(19):3819-3824).

As described in more detail in the examples below, constructs are described which provide for enhanced expression of DNA sequences in plant plastids. Promoter/ribosome binding site sequences are employed to direct expression in plant plastids are promoter sequences of the 16S ribosomal RNA operon (P*rrn*) which are linked to a ribosome binding site (RBS) derived from the T7 bacteriophage gene 10 leader sequence (G10L). DNA sequences expressed under the regulatory control of the P*rrn*/G10L sequence show a significantly higher level of protein expression than those levels obtained under the control of other promoter/RBS combinations, while expression of mRNA may or may not be higher in these plants.

In the examples below, nucleic acid sequences encoding CP4 EPSP synthase (USPN 5,633,435) are placed into expression constructs for expression of EPSP synthase enzyme from the plant plastid. The constructs prepared utilize a ribosome binding site designed after the T7 bacteriophage gene 10 leader (G10L) to increase the expression of the nucleic acid sequences in the plant plastid.

Plastid expression constructs encoding for the expression of EPSPS are introduced via a chloroplast transformation vector.

Tobacco lines containing the native encoding sequence to the EPSPS enzyme expressed in plastids under the control of the P*rrn*/G10L promoter/ribosome binding site sequence demonstrate a significantly higher level of protein expression than those levels obtained from EPSPS expressed under the control of the P*rrn*/rbcL RBS sequence. However, EPSPS mRNA is expressed at a higher level in plants expressing CP4 EPSPS from the plastid under the control of the P*rrn*/rbcL(RBS). These results indicate that translation from transcripts containing the T7 bacteriophage gene 10 ribosome binding site is more efficient. In addition, protein expression levels of EPSPS obtained from transplastomic tobacco lines expressing EPSPS under the control of the P*rrn*/G10L RBS provide for a high level of glyphosate tolerance.

Furthermore, transplastomic tobacco lines transformed to express hGH under the control of the P*rrn*/G1 0L promoter/ribosome binding site sequence demonstrate a significantly higher level of protein expression than those levels obtained from hGH expressed under the control of the PpsbA promoter/RBS sequence.

Increases in protein expression levels of at least approximately 200 fold may be obtained from constructs utilizing P*rrn*/G10L ribosome binding site for expression of EPSPS over the expression levels obtained from other promoter/RBS combinations for plastid expression. In addition, protein levels obtained from plastid expression constructs utilizing the P*rrn*/G10L promoter/RBS sequence may accumulate 50 to 3500 fold higher levels than from nuclear expression constructs. Thus, inclusion of the G10L ribosome binding site in plastid expression constructs may find use for increasing the levels of protein expression from plant plastids.

Furthermore, the constructs of the present invention may also include sequences to target the expressed protein to a particular suborganellar region, for example, the thylakoid lumen of the chloroplast. For example, as described in the examples below, a nucleotide sequence encoding a peptide from the plastid genome cytochrome *f* targets the expressed aprotinin protein to the thylakoid membrane. Such targeting of expressed proteins may provide for a compartmentalization of the protein allowing for increased oxidative stability and proper protein folding.

Thus, the constructs and methods of the present invention provide a means for obtaining transplastomic plants with high level tolerance of herbicides. High levels of tolerance include tolerance of vegetative tissue when amounts of greater than about 454 g (about 16 oz)/acre glyphosate are applied, preferably greater than about 907 g (about 32 oz)/acre, more preferably greater than about 1.81 kg (about 64 oz)/acre, most preferably greater than about 3.639 g (about 128 oz)/acre. Furthermore, high levels of tolerance can also include tolerance of reproductive tissues when amounts of greater than about 454 g (about 16 oz)/acre glyphosate are applied, preferably greater than about 907 g (about 32 oz)/acre, most preferably greater than about 1.81 kg (about 64 oz)/acre.

The invention now being generally described, it will be more readily understood by reference to the following examples.

### EXAMPLES

### Example 1 Expression Constructs

Constructs and methods for use in transforming the plastids of higher plants are described in Zoubenko *et al.* (*Nuc Acid Res* (1994) 22(19):3819-3824), Svab *et al.* (*Proc. Natl. Acad. Sci.*(1990) 87:8526-8530 and *Proc. Natl. Acad. Sci*.(1993) 90:913-917) and Staub *et al.* (*EMBO J.* (1993) 12:601-606). Constructs and methods for use in transforming plastids of higher plants to express DNA sequences under the control of a nuclearly encoded, plastid targeted T7 polymerase are described in U.S. Patent Number 5,576,198. The complete DNA sequences of the plastid genome of tobacco are reported by Shinozaki *et al*. (*EMBO J.* (1986) 5:2043-2049). All plastid DNA references in the following description are to the nucleotide number from tobacco.

The complete nucleotide sequence encoding the tobacco cytochrome *f* (*pet*A) is described in Bassham et al, (1991) *J Biol Chem* 266:23606-23610 and Konishi *et al*. (1993) *Plant Cell Physiol* 34:1081-1087.

### 1A. Promoter/Ribosome Binding Site Sequences

The promoter region of the plastid 16S ribosomal RNA operon (P*rrn*) is linked to a synthetic ribosome binding site (RBS) patterned on the plastid rbcL gene leader to create the P*rrn*/rbcLRBS fragment. The P*rrn*/rbcLRBS sequence is as described in Svab *et al.* (1993, *supra*) for the Prrn/rbcL(S) fragment.

The promoter region of the plastid psbA promoter (PpsbA) and terminator sequences (TpsbA) are described in Staub *et al.* (1993, *EMBO J.,* 12, 601-606).

The P*rrn*/G10L sequence was constructed by annealing two oligonucleotide sequences, T7lead1 and T7lead2 (Table 1), to create the G10L plastid ribosome binding site (Figure 1). The G10L sequence was ligated to the 3' terminus of the P*rrn* promoter sequence as an *Eco*RI/*Nco*I fragment to create the P*rrn*/G 10L sequence.

**Table 1**

| | |
|---|---|
| **T7lead1** | 5'-AAT TGT AGA AAT AAT TTT GTT TAA CTT TAA GAA GGA GAT ATA CC-3' |
| **T7lead2** | 5'-CAT GGG TAT ATC TCC TTC TTA AAG TTA AAC AAA ATT ATT TCT AC-3' |

Chimeric genes are preferably inserted into the expression vector to direct their transcription from the P*rrn* promoter. Thus, in the plastid genome, chimeric genes are transcribed from the P*rrn*/RBS promoter, or the Prrn/G10L promoter in the plant plastid. The nucleic acid sequence of the Prrn/G10L fusion is provided in figure 6.

### 1B. CP4 EPSPS Plastid Expression Constructs

A plastid expression vector pMON30117 is constructed from a precursor vector pPRV111B (Zoubenko, *et al*. 1994, *supra*, GenBank accession U12813). The vector pMON30117 carries a multiple cloning site for insertion of a passenger gene in a P*rrn*/rbcLRBS/T*rps*16 expression cassette. The P*rrn*/rbcLRBS sequence is cloned into pPRV111B vector as an *Eco*RI/*Nco*I fragment, and the terminator region from the plastid *rps*16 gene(T*rps*16) is cloned 3' of the Prrn promoter as a *Hind*III/*Nco*I fragment. The T*rps*16 fragment comprises the *rps*16 gene 3'-regulatory region from nucleotides 5,087 to 4,939 in the tobacco plasmid DNA.

The pPRV111B backbone of the vector pMON30117 contains a marker gene, *aad*A, for selection on spectinomycin and streptomycin, and *rps* 7/12 for the integration, by homologous recombination, of the passenger DNA into *trn*V-*rps*7/12 intergenic region.

A nuclear expression construct, pMON10154, was prepared as a control for integration into plants by Agrobacterium-mediated transformation. In this construct, the CP4 native gene is expressed from the constitutive Figwort Mosaic Virus promoter and the Petunia HSP70 leader, and has the E9 terminator. Targeting to plastids is by the chloroplast transit peptide of the Petunia EPSPS translationally fused to the N-terminus of the CP4 gene.

The plastid expression construct pMON30118 was prepared by cloning the native CP4 EPSPS gene fused with the N-terminal five (5) amino acids from the plastid *rbc*L (described in Svab *et al*., 1993 *supra*) gene as an *Nco*I/*Sma*I fragment into the multiple cloning site of the vector pMON30117.

The plastid expression construct pMON30123 is essentially the same as pMON30118 with the exception of the deletion of the N-terminal five (5) amino acids from the plastid *rbc*L.

The plastid expression construct pMON30130 was created by replacing the native CP4 EPSPS of pMON30123, with a synthetic CP4 gene. This construct also lacks the N-terminal 5 amino acid fusion from the plastid rbcL gene.

The plastid expression construct pMON38773 was constructed by replacing the P*rrn*/RBS sequence of pMON30123 with the P*rrn*/G10L promoter sequence described above. The EPSPS DNA sequence of pMON38773 also lacks the N-terminal 5 amino acid fusion from the plastid rbcL gene.

A plastid expression construct, pMON38766 was constructed using the promoter from T7 phage gene 10 (P-T7), including G10L, CP4 (native) gene coding region, and the terminator sequence from plastid rps16 gene (Trps16).

A plastid expression construct, pMON38797 was constructed using the promoter from T7 phage gene 10 (P-T7), including G10L, CP4 (synthetic) gene coding region, terminator from plastid rps16 gene (Trps16).

A plastid expression construct, pMON38798 was constructed using the promoter of the 16SrDNA operon (Prrn), G10L, CP4 (synthetic) gene coding region, terminator from plastid rps16 gene (Trps16).

A plastid expression construct, pMON38793 was constructed using the promoter of the 16SrDNA operon (Prrn), a synthetic ribosome binding site (RBS) patterned from the plastid rbcL gene, the glyphosate tolerant Petunia EPSP synthase gene (P-EPSPS, Padgette, *et al.*(1987) *Arch. Biochem, Biophys*. 258:564-573) carrying the mutation Glycine to Alanine at amino acid position 101, terminator from plastid rps16 gene (Trps16).

A plastid expression construct, pMON38796 was constructed using the promoter of the 16SrDNA operon (Prrn), synthetic ribosome binding site (RBS) patterned from the plastid rbcL gene, the glyphosate tolerant Achromobacter (strain LBAA) EPSP synthase gene (U.S. Patent Number 5,627,061, the entirety of which is incorporated herein by reference)carrying the mutation Glycine to Alanine at amino acid position 100 (G100A), terminator from plastid rps 16 gene (Trps 16).

A plastid expression construct, pMON45204, was constructed using the promoter of the 16SrDNA operon (Prrn) with the G10L, the glyphosate tolerant Pseudomonas (strain LBAA) EPSP synthase gene carrying the mutation Glycine to Alanine at amino acid position 100 (G100A), terminator from plastid rps16 gene (Trps16).

A plastid expression construct, pMON45201, was constructed using the promoter of the 16SrDNA operon (Prrn), synthetic ribosome binding site (RBS) patterned from the plastid rbcL gene, wild-type glyphosate tolerant Bacillus subtilis aroE (EPSPS)(U.S. Patent Number 5,627,061) gene, terminator from plastid rps16 gene (Trps16).

A plastid expression construct, pMON45259, was constructed using the promoter of the 16SrDNA operon (Prrn) with the G10L sequence functionally associated with the nucleic acid sequence encoding the synthetic CP4 protein having an additional sequence at the N-terminus encoding the first 14 amino acids of the green fluorescent protein (GFP) (GKGEELFTGVVPSM). The sequence encoding the 14 amino acid GFP fusion begins at the glycine in the second position of the protein. The construct also contains the rps 16 terminator.

Another plastid expression construct, pMON49218, was constructed to express the synthetic CP4 sequence with the 14 amino acid GFP fusion from the promoter region of the 16SrDNA operon having the nuclear-encoded RNA polymerase region (PrrnPEP+NEP), and the terminator region from the plastid rps16 gene. The DNA sequence of the Prrn/NEP/G10L::14aaGFP fusion is provided in figure 7.

### Example 2 Plant Transformation

### 2A. Nuclear Transformation

Tobacco plants transformed to express the constructs pWRG4744 and pWRG4747 in the nucleus of a plant cell may be obtained as desribed by Horsch *et al.* (*Science* (1985) 227:1229-1232).

### 2B. Plastid Transformation

Tobacco plastids are transformed by particle gun delivery of microprojectiles as described by Svab and Maliga (*Proc. Natl. Acad. Sci.* (1993) 90:913-917), and described here.

Dark green, round leaves are cut, preferably from the middle of the shoots, from 3-6 week old *Nicotiana tabacum* cv. Havana which have been maintained *in vitro* on hormone free MS medium (Murashige and Skoog, (1962) *Physiol Plant*. 15, 473-497) supplemented with B5 vitamins in Phytatrays or sundae cups with a 16 hour photoperiod at 24°C. Each cut leaf is then placed adaxial side up on sterile filter paper over tobacco shoot regeneration medium (TS0 medium: MS salts, 1mg/l *N*⁶-benzyladenine, 0.1mg/l 1-naphthaleneacetic acid, 1 mg/l thiamine, 100mg/l inositol, 7g/l agar pH 5.8 and 30g/l sucrose). Leaves are preferably placed in the center of the plate with as much contact with the medium as possible. The plates are preferably prepared immediately prior to use, but may be prepared up to a day before transformation by particle bombardment by wrapping in plastic bags and storing at 24°C overnight.

Tungsten or gold particles are sterilized for use as microcarriers in bombardment experiments. Particles (50mg) are sterilized with I ml of 100% ethanol, and stored at -20°C or -80°C. Immediately prior to use, particles are sedimented by centrifugation, washed with 2 to 3 washes of 1 ml sterile deionised distilled water, vortexed and centrifuged between each wash. Washed particles are resuspended in 500 µl 50% glycerol.

Sterilized particles are coated with DNA for transformation. Twenty-five micoliter aliquots of sterilized particles are added to a 1.5 ml microfuge tube, and 5 µg of DNA of interest is added and mix by tapping. Thirty-five microliters of a freshly prepared solution of 1.8M CaCl₂ and 30 mM spermidine is added to the particle/DNA mixture, mixed gently, and incubated at room temperature for 20 minutes. The coated particles are sedimented by centrifuging briefly. The particles are washed twice by adding 200µl 70% ethanol, mixing gently, and centifuging briefly. The coated particles are resuspended in 50µl of 100% ethanol and mixed gently. Five to ten microliters of coated particles are used for each bombardment.

Transformation by particle bombardment is carried out using the PDS 1000 Helium gun (Bio Rad, Richmond, CA) using a modified protocol described by the manufacturer.

Plates containing the leaf samples are placed on the second shelf from the bottom of the vacuum chamber and bombarded using the 1100 p.s.i. rupture disk. After bombardment, petriplates containing the leaf samples are wrapped in plastic bags and incubated at 24°C for 48 hours.

After incubation, bombarded leaves are cut into approximately 0.5 cm² pieces and placed abaxial side up on TSO medium supplemented with 500 µg/ml spectinomycin. After 3 to 4 weeks on the selection medium, small, green spectinomycin resistant shoots will appear on the leaf tissue. These shoots will continue to grow on spectinomycin containing medium and are referred to as primary putative transformants.

When the primary putative transformants have developed 2 to 3 leaves, 2 small pieces (approximately 0.5 cm²) are cut from each leaf and used for either selection or for a second round of shoot regeneration. One piece is placed abaxial side up on plates containing TSO medium supplemented with 500 µg/ml spectinomycin, and the other piece is placed abaxial side up on TSO medium supplemented with 500 µg/ml each of spectinomycin and streptomycin. Positive transformants are identified as the shoots which form green callus on the TSO medium containing spectinomycin and streptomycin.

After 3 to 4 weeks, the tissue placed on TSO medium containing only spectinomycin, which has been identified as positive on the TSO medium with spectinomycin and streptomycin, will develop green shoots. Two to four shoots of each positive transformant are selected and transferred to TSO medium supplemented with 500 µg/ml spectinomycin for generation of roots. Southern analysis is performed on 2 shoots to confirm homoplasmy as described below. Shoots from homoplasmic events are transferred to the greenhouse for seed production, while transformants which are not homoplasmic are sent through a second round or regeneration on TSO medium with 500 µg/ml spectinomycin to attain homoplasmy.

### Example 3 Analysis of Transplastomic Tobacco Plants Transformed with Herbicide Tolerance Constructs

### 3A. Southern Analysis

Transformed plants selected for marker *aad*A marker gene expression are analyzed to determine whether the entire plastid content of the plant has been transformed (homoplastic transformants). Typically, following two rounds of shoot formation and spectinomycin selection, approximately 50% of the transgenic plantlets which are analyzed are homoplastic, as determined by Southern blot analysis of plastid DNA. Homoplasmic plantlets are selected for further cultivation.

Genomic DNA is isolated from transformed tobacco plants, electrophoresed, and transferred to filters as described in Svab *et al*. ((1993), *Proc Natl Acad Sci*, 90:913-917).

Homoplasmic tobacco plants transformed to express CP4 EPSPS in plastids were identified using a probe prepared from a 2.4 kb *Eco*RI/*Eco*RV fragment from the vector pOVZ2 (similar to pOVZ15 described in Zoubenko, *et al.* 1994, *supra*). The 2.4 kb probe fragment encompasses part of the targeting sequence.

Results of the Southern hybridizations identified 3 homoplasmic lines from tobacco transformed with the constructs pMON30123 and pMON30130 and 1 line from tobacco transformed with pMON38773 for further analysis.

The complete disappearance of the 3.27 Kb native tobacco *Bam*HI fragment in the lines 30123-19-1A, 30123-23-2A, 30123-18-1B, 30130-51-2A, 30130-51-2P. 30130-57-1P, and 38773-6 with a probe covering the region of integration, and the appearance of expected sized bands for the inserted DNA fragments in those transformants. 5.14 kb and 0.9 kb, establishes that the transformed plants are homoplasmic for the intended constructs.

Results of the Southern hybridizations identified 3 homoplasmic lines from tobacco transformed with pCGN5177, lines 74-1B-P, 74-2 and 74-7.

Transplastomic 5175 and 6114 tobacco lines were analyzed by Sourthern hybridization for homoplasmy as described above. Results of the Southern hybridizations identified 4 homoplasmic lines from tobacco transformed with pCGN6114.

Results from hybridizations of 5175 transplastomic tobacco lines identified one line, 76-4A-F, as homoplasmic, and a second line as 95% homoplasmic.

### 3B. Northern Analysis

In order to determine the level of transcription of the EPSPS, mRNA expressed in the transplastomic tobacco plants, Northern blot hybridizations were performed with total RNA isolated from each of the lines identified. Total RNA was isolated using TRIzol reagent (Gibco-BRL Life Technologies, Gaithersburg, MD) according to the manufacturers protocol. Total RNA, 2µg, was separated on a denaturing agarose gel and transferred to nylon membrane (Maniatis *et al*., 1989, *supra*). Radioactive probes for hybridizations were prepared using random primer labeled (using Random Primer labeling kit from Boehringer Mannheim) CP4 EPSPS fragments and hybridizations were carried out in 2x SSPE (Maniatis, *et al*., 1989,*supra*), at 60°C. Filters were stripped and reprobed with a plastid 16S ribosomal RNA gene probe (from pPRV112A. Zoubenko, *et al*., 1994, *supra*) to confirm homogenous loading of RNA on the filter.

Results of the Northern hybridizations performed with EPSPS probes demonstrate that all seven (7) lines examined express CP4 EPSPS mRNA. Hybridizations performed with the 16S ribosome probe confirm that denaturing gels were loaded with similar amounts of total RNA for each sample. Furthermore, transplastomic tobacco lines expressing EPSPS from the P*rrn*/rbcL(RBS) (pMON30123) regulatory elements express EPSPS mRNA to higher levels than tobacco plants homoplasmic for EPSPS controlled by the P*rrn*/G10L (pMON38773) promoter/RBS sequences.

### 3C. Western Blot Analysis of Tobacco CP4 EPSPS

To determine the expression of the EPSPS Western blot analysis was performed on a single line from each construct, pMON30123, pMON30130, and pMON38773.

Total soluble protein was extracted from frozen leaf tissue by grinding 250 mg tissue in 250µl of PBS buffer (1 mM KH₂PO₄, Na₂HPO₄, 0.137M NaCl, 2.7 mM KCl pH 7.0) containing protease inhibitors. The homogenate is centrifuged for 5 minutes, and the supernatant is transferred to a fresh tube. The concentration of the protein in the supernatant is determined using a protein concentration assay (BioRad, Richmond, CA).

Extracted total protein is electrophoresed on a 4-20% SDS-PAGE gel (Sigma, St Louis, MO), and transferred to PVDF membrane in 1x SDS-PAGE buffer (Maniatis *et al*. 1989, Cold Spring Harbor Press). Standards of quantitated purified CP4 EPSPS protein were used to quantify the expression of the CP4 EPSPS as expressed in the plant plastid.

Western hybridizations are performed as described in Staub and Maliga (1993) *EMBO Journal*, 12(2) 601-606, except using antibodies raised to EPSPS. PVDF membranes containing the transferred electrophoresed protein were incubated in a blocking solution of PBS buffer containing 0.05% Tween-20 (PBS-T) and 5% milk overnight at 4°C. The membranes are then incubated in a solution of PBS-T containing I % milk and a primary antibody raised in goats to the CP4 EPSPS for 2 hours at room temperature. The membranes are washed three times in a solution of PBS-T containing 0.1% milk, each wash for 5 minutes at room temperature. The membranes are then incubated in a solution of PBS-T containing 1% milk and sheep anti-goat antibody for. 1 hour at room temperature, and washed again in PBS-T containing 0.1% milk, three times for 10 minutes at room temperature. A final wash using only PBS-T is performed before developing the membranes using a nonradioactive detection kit (ECL, Amersham).

**Table 2**

| Construct Number | % Total Soluble Protein |
|---|---|
| pMON30123 | 0.001 |
| pMON30130 | 0.002 |
| pMON38773 | 0.2 |
| pMON38798 | 0.2 |
| pMON45259 | >12.0. |
| pMON49218 | >12.0 |

The results listed in Table 2 demonstrate that significant increases in the level of EPSPS protein may be obtained from plants transformed to express EPSPS from the P*rrn*/G10L promoter. These results demonstrate that EPSPS expression driven by the P*rrn*/rbcLRBS regulatory sequences may produce approximately 0.001% of the total soluble protein as EPSPS, while in plants expressing EPSPS from the P*rrn*/G10L regulatory sequences express 0.2% of the total soluble protein as EPSPS. Subsequent lines have demonstrated total soluble protein of about 1% EPSPS when expressed from the P*rrn*/G10L regulatory sequences. These results, taken together with the results of the Northern hybridizations above, indicate that more efficient translation may be obtained from the G10L ribosome binding site.

Furthermore, plastid expression constructs containing the N-terminal 14 amino acid from GFP demonstrated high levels of protein expression. Transplastomic lines containing either pMON45259 or pMON49218 demonstrated total soluble protein of greater than 12% CP4 EPSPS.

### 3D. Analysis of EPSPS Enzyme Activity

The EPSPS enzyme activity in transplastomic tobacco plants containing the plastid expression vector pMON38773 was determined using a high pressure liquid chromotography (HPLC) assay. Methods for the analysis of EPSPS enzyme activity are described in Padgette *et al*. (*J. Biol. Chem.* (1988)263:1798-1802 and *Arch. Biochem. Biophys.* (1987)258:564-573) and Wibbenmeyer *et al.* (*Biochem. Biophys. Res. Commun.* (1988)153:760-766). The results are summarized in Table 3 below.

**Table 3**

| **Nuclear Enzymatic Activity Range** | **Nuclear % Total Plants In Range** | **Chloroplast 38773-6** |
|---|---|---|
| 1-3.7 µmol/mg | 1% | |
| >0.1 µmol/mg | 16% | |
| >10 nmol/mg | 55% | 16.39 nmol/mg |
| >1 nmol/mg | 32% | |
| 0 nmol/mg | 3% | |

These results demonstrate that EPSPS expression in plastids produces active EPSPS enzyme.

### 3E. Analysis for Glyphosate Tolerance

A transplastomic tobacco line homoplasmic for the construct pMON38773 was tested *in vitro* to determine the highest level of glyphosate tolerance. Explant tissue was prepared from leaf pieces of nontransgenic wild type tobacco control, *Havanna*, plants and the homoplasmic tobacco line 38773-6 and cultured for regeneration of shoots on TSO medium (described above) supplemented with glyphosate levels of 50µM, 75µM, 100µM, 150µM and 200µM. The results are summarized in Table 4 below. The number of explants producing shoots was determined at 3 weeks and 6 weeks after explant preparation and culturing on glyphosate containing medium.

**Table 4**

| **Glyphosate Level (µM)** | **Total Number Explants** | **Number Regenerating 3 Weeks** | **Number Regenerating 6 Weeks** | **% Explant Regeneration** |
|---|---|---|---|---|
| | | | | |

| **Wild Type** | | | | |
|---|---|---|---|---|
| 50 | 10 | 0 | 0 | 0 |
| 75 | 10 | 0 | 0 | 0 |
| 100 | 10 | 0 | 0 | 0 |
| 150 | 10 | 0 | 0 | 0 |
| 200 | 10 | 0 | 0 | 0 |
| | | | | |

| **38773-6** | | | | |
|---|---|---|---|---|
| 50 | 8 | 5 | 8 | 100 |
| 75 | 18 | 14 | 18 | 100 |
| 100 | 17 | 12 | 15 | 88 |
| 150 | 18 | 10 | 16 | 89 |
| 200 | 16 | 8 | 15 | 86 |

The above results demonstrate that at all levels of glyphosate examined, shoots regenerated from explants prepared from a tobacco line homoplasmic for pMON38773, while no shoots regenerated from explants prepared from nontransfonmed control plants. These results suggest that tobacco plants expressing EPSPS in plastids demonstrate tolerance to glyphosate levels of at least 200µM.

Additional transplastomic lines were tested *in vitro* for glyphosate tolerance as described above. The results are shown in Table 5

**TABLE 5**

| **Summary of tobacco plastid transformation experiments with various constructs containing EPSPS genes.** | | |
|---|---|---|
| Construct | Spec/strep (+) | No. of shoots Gly 50 uM(+) |
| pMON38766 (Wild) | 1 | 0 |
| pMON38766 (T7) | 6 | 0 |
| pMON38773 (Wild) | 9 | 5(1) |
| pMON38797 (Wild) | 2 | 0 |
| pMON38798 | 6 | 6 |
| pMON38793 | 8 | 0 |
| pMON38796 | 4 | 0 |
| pMON45201 | 9 | 3 |
| pMON45204 | 12 | * |
| (No. of shoots positive at 1 mM glyphosate) | | |

These results demonstrate that these transplastomic lines show tolerance to glyphosate. The numbers in parentheses are the number of shoots resistant to selection at 1 mM glyphosate. Thus, as can be seen in table 5, tobacco lines are generated that are tolerant of selection at 1 mM glyphosate.

Homoplasmic tobacco plants of the line 38773-6 are sprayed with glyphosate using a track sprayer at concentrations corresponding to 0oz/acre 454 g, (16oz)/acre, 907 g (32oz)/acre and 1.81 kg (60oz)/acre to test for whole plant tolerance. Plant height was measured before and after spraying with glyphosate. The vegetative injury data was collected two weeks after spraying, while the reproductive injury data was collected at plant maturity.

Initial results indicate that homoplasmic tobacco lines sprayed are tolerant of glyphosate at the concentration of 454 g (16oz)/acre as demonstrated in the vegetative tissue injury (Table 6). As can be seen in Table 5 transplastomic lines were generated which demonstrated a good level of glyphosate tolerance at 907 g (32oz)/Acre. In subsequent experiments with additional transformed lines, transplastomic lines have shown tolerance to glyphosate at a level of 1.81 kg (64oz)/Acre.

Tolerance is characterized by the continued growth and greening of tissues sprayed with glyphosate. However, as the concentration of glyphosate applied increased, there was a corresponding increase in the level of vegetative injury. In contrast, nontransformed control plants which were highly susceptible to glyphosate concentrations as low as 454 g (16 oz)/Acre.

**Table 6**

| Plant No. | Construct | Roundup rate (oz/A) | Plant height (cm) before spray | Plant height (cm) after spray | Vegetative injury | Fertility rating |
|---|---|---|---|---|---|---|
| 1 | 38773 | 0 | 12.2 | 30.5 | 0 | 0 |
| 2 | 38773 | 0 | 13.6 | 34.0 | 0 | 0 |
| 3 | 38773 | 0 | 8.6 | 23.8 | 0 | 0 |
| 4 | 38773 | 0 | 8.6 | 26.2 | 0 | 0 |
| 5 | 38773 | 0 | 7.8 | 28.8 | 0 | 0 |
| 6 | 38773 | 0 | 12.8 | 31.5 | 0 | 0 |
| 7 | 38773 | 0 | 12.2 | 31.6 | 0 | 0 |
| 8 | 38773 | 0 | 11.6 | 35.5 | 0 | 0 |
| 9 | 38773 | 16 | 9.0 | 29.0 | 1 | 0 |
| 10 | 38773 | 16 | 14.4 | 31.0 | 0 | 0 |
| 11 | 38773 | 16 | 13.4 | 32.0 | 0 | 0 |
| 12 | 38773 | 16 | 13.2 | 30.0 | 0 | 0 |
| 13 | 38773 | 16 | 14.2 | 30.5 | 0 | 1 |
| 14 | 38773 | 16 | 14.0 | 33.0 | 0 | 0 |
| 15 | 38773 | 16 | 13.2 | 30.2 | 0 | 0 |
| 16 | 38773 | 16 | 14.9 | 30.4 | 0 | 0 |
| 17 | 38773 | 32 | 12.0 | 26.5 | 2 | 4 |
| 18 | 38773 | 32 | 11.6 | 25.4 | 1 | 1 |
| 19 | 38773 | 32 | 9.4 | 22.0 | 1 | 3 |
| 20 | 38773 | 32 | 11.2 | 23.0 | 2 | 4 |
| 21 | 38773 | 32 | 13.8 | 25.8 | 1 | 2 |
| 22 | 38773 | 32 | 12.4 | 23.0 | 1 | 4 |
| 23 | 38773 | 32 | 10.2 | 19.0 | 2 | 4 |
| 24 | 38773 | 32 | 13.8 | 23.2 | 2 | 3 |
| 26 | 38773 | 64 | 11.8 | 20.0 | 2 | 5 |
| 27 | 38773 | 64 | 13.0 | 22.0 | 2 | 5 |
| 28 | 38773 | 64 | 12.2 | 18.0 | 3 | 5 |
| 29 | 38773 | 64 | 15.8 | 23.0 | 2 | 5 |
| 30 | 38773 | 64 | 10.4 | 17.5 | 2 | 5 |
| 32 | 38773 | 64 | 15.0 | 18.5 | 2 | 5 |
| 33 | 38773 | 64 | 13.8 | 21.8 | 2 | 5 |
| 34 | 38773 | 64 | 13.6 | 19.0 | 3 | 5 |
| 35 | 38773 | 64 | 10.8 | 16.0 | 3 | 5 |
| 36 | Wild type | 0 | 21.0 | 40.6 | 0 | 0 |
| 37 | Wild type | 0 | 16.0 | 38.0 | 0 | 0 |
| 38 | Wild type | 0 | 15.0 | 34.6 | 0 | 0 |
| 39 | Wild type | 0 | 17.6 | 32.2 | 0 | 0 |
| 40 | Wild type | 0 | 15.0 | 31.6 | 0 | 0 |
| 41 | Wild type | 0 | 14.0 | 32.0 | 0 | 0 |
| 42 | Wild type | 16 | 10.0 | 11.8 | 3 | 5 |
| 43 | Wild type | 16 | 8.0 | 10.0 | 3 | 5 |
| 44 | Wild type | 16 | 8.6 | 11.0 | 3 | 5 |
| 45 | Wild type | 16 | 8.0 | 14.0 | 3 | 5 |
| 46 | Wild type | 16 | 9.8 | 11.0 | 3 | 5 |
| 47 | Wild type | 16 | 10.4 | 14.0 | 3 | 5 |
| 48 | Wild type | 32 | 10.8 | 13.2 | 3 | 5 |
| 49 | Wild type | 32 | 9.0 | 13.0 | 3 | 5 |
| 50 | Wild type | 32 | 8.0 | 10.2 | 3 | 5 |
| 51 | Wild type | 32 | 11.0 | 14.0 | 4 | 5 |
| 52 | Wild type | 32 | 9.8 | 13.0 | 3 | 5 |
| 53 | Wild type | 32 | 8.0 | 10.8 | 4 | 5 |
| 54 | Wild type | 64 | 7.5 | 8.6 | 4 | 5 |
| 55 | Wild type | 64 | 11.2 | 12.5 | 4 | 5 |
| 56 | Wild type | 64 | 10.2 | 12.8 | .4 | 5 |
| 57 | Wild type | 64 | 11.5 | 13.0 | 4 | 5 |
| 58 | Wild type | 64 | 13.0 | 15.0 | 4 | 5 |
| 59 | Wild type | 64 | 9.8 | 11.2 | 4 | 5 |

| | | | | | | |
|---|---|---|---|---|---|---|
| Vegetative injuries: 0=normal plant I=slight chlorosis of new leaves and stunting 2=severe chlorosis of new leaves, malformation of new leaves, and severe stunting 3=dying plant 4=dead plant Fertility ratings: 0 = Fertile, no delay in maturity, lots of seed 1 = Some abortion, slight delay in seed set, seed 2 = Significant abortion, significant delay in seed set, some seed 3 = Very severe abortion, immature seed pots, a few seed 4 = malformed flowers; if flowered, extreme delay in flowering and no seed produced 5=dead plant 16 oz/A =̂ 454 g/acre; 32 oz/A =̂ 907 g/acre, 69 oz/A =̂ 1.81 kg/acre | | | | | | |

In addition, other transplastomic lines were analyzed for tolerance to spraying with various levels of glyphosate as described above. Specific activity is measured as the amount of exogenously added Phosphoenol pyruvate (PEP) converted to Shikimate-3-phosphate (S3P) per unit protein in the plant extract. Addition of glyphosate tests sensitivity of the EPSPS enzyme to glyphosate. The results are summarized in Table 7.

**Table 7**

| LINE | % TOTAL SOLUBLE PROTEIN | SPECIFIC ACTIVITY (nmol/min/mg) No gly | SPECIFIC ACTIVITY (nmol/min/mg) +1 mM gly | Vegetative tolerance (oz/acre) | Reproductive tolerance (oz/acre) |
|---|---|---|---|---|---|
| Wild-type | | 3.4 | 0 | 0 | 0 |
| pMON10154 | 0.04 | 19.0 | 18.4 | 128 | 64 |
| pMON45201 | - | 301.6 | 221.2 | 32 | 32 |
| pMON45204 | - | 339.9 | 371.8 | 128 | 64 |
| pMON30123 | 0.001 | 4.0 | 0 | 0 | 0 |
| pMON30130 | 0.002 | 6.2 | 0 | 0 | 0 |
| pMON38773 | 0.2 | 16.7 | 6.7 | 32 | 16 |
| pMON38798 | 0.2 | 17.2 | 14.7 | 32 | 16 |
| pMON45259 | >12.0 | - | - | 128 | 64 |
| pMON49218 | >12.0 | - | - | 128 | 64 |

| | | | | | |
|---|---|---|---|---|---|
| 32 oz/acre =̂ 907 g/acre; 128 oz/acre =̂ 3.63 kg/acre; 64 oz/acre =̂ 1.81 kg/acre 16 oz/acre =̂ 454 g/acre | | | | | |

These data demonstrate that high levels of glyphosate tolerance can be obtained in transplastomic plants expressing various EPSPS sequences. In particular, lines pMON45204, pMON45259, and pMON49218 provide tolerance to glyphosate applied at levels of at least 3.63 kg (128 oz)/acre on vegetative tissues, and at least 1.81 kg (64 oz)/acre on reproductive tissues.

Furthermore, constructs pMON42259 and pMON49218 provide for high level expression of CP4 EPSPS from plant plastids transformed with these constructs. In particular, expression levels of greater than about 12 percent total soluble protein are obtained in constructs employing sequences encoding the first 14 amino acids of GFP fused to the N-terminus of CP4.

## Claims

1. A construct comprising the following components in the 5' to 3' direction of transcription:
(a) a promoter sequence of the 16S ribosomal RNA operon (Prrn) functional in a plant plastid;
(b) a ribosome binding site of the T7 bacteriophage polymerase gene 10 leader joined to said promoter component (a);
(c) a DNA sequence encoding the *Agrobacterium* CP4 5-enolpyruvylshikimate-3-phosphate synthase (EPSPS) which is capable of conferring tolerance in a plant cell to at least the herbicide compound glyphosate when said DNA sequence is transcribed in plastids of said plant cell; and
(d) a transcription termination region.

2. The construct according to claim 1, wherein said construct further comprises (e) a gene encoding a selectable marker for selection of plant cells comprising a plastid expressing said marker and (f) DNA regions of homology to the genome of said plastid, wherein said regions of homology in (f) flank components (a), (b), (c), (d) and (e).

3. The construct according to claim 1 or 2, which further contains a sequence encoding the N-terminal 14 amino acids from GFP at the N-terminus of the protein encoded by (c).

4. A plant cell plastid containing the construct according to any one of claims 1 to 3.

5. A plant, plant seed, plant cell or progeny thereof containing a plant plastid according to claim 4.

6. A method for producing tolerance of a herbicide in a plant cell wherein said method comprises transforming plastids of said plant cell with a construct as defined in any one of claims 1 to 3, and growing plant cells comprising said transformed plastids under conditions wherein said DNA sequence is transcribed whereby plant cells containing said plant plastids are rendered tolerant to applications of glyphosate.

7. The method according to claim 6, wherein said construct further comprises a second DNA sequence of interest.

8. The method according to claim 7, wherein said second DNA sequence comprises a gene expressed from a promoter independent from said promoter (a).

9. The method according to claim 7, wherein said second DNA sequence comprises a gene expressed from said promoter (a) as a polycistronic message with said DNA sequence in (c).

10. The method according to claim 7, wherein said second DNA sequence comprises a gene other than a gene capable of conferring in a plant cell tolerance to said herbicide compound.

11. The method according to claim 7, wherein said second DNA sequence comprises a gene capable of conferring in a plant cell tolerance to a second herbicide compound.

12. A herbicide tolerant plant cell obtainable by the method of claim 6.

13. A plant, plant seed or plant part comprising a plant cell according to claim 12.

14. The plant cell according to claim 12 which comprises greater than about 0.01% of total soluble proteins as a protein expressed from said herbicide-tolerance gene.

15. The plant cell according to claim 12 which comprises greater than about 0.1%, preferably greater than about 0.2% of total soluble proteins as a protein expressed from said herbicide-tolerance gene.

16. The plant cell according to claim 12 which has a construct as defined in claim 3 and comprises 1% or more, preferably 12% or more of total soluble proteins as a protein expressed from said herbicide-tolerance gene.

17. A plant, plant seed or plant part comprising a plant cell according to claim 16.

18. A method wherein herbicide-tolerance obtainable by the method of claim 6 is used to select cells transformed by said construct from non-transformed cells.

19. The method according to claim 18, wherein selection for said herbicide tolerance is made on media containing glyphosate at a concentration from at least about 50 µM to about 200 µM.

20. The method according to claim 19, wherein selection for said herbicide tolerance is made on media containing glyphosate at a concentration from about 1 mM.

21. A plant cell obtainable by the method of claim 18.

## Patentansprüche

1. Konstrukt, das die folgenden Komponenten in 5'→3'-Transcriptionsrichtung umfasst:
(a) Promotorsequenz des 16S-ribosomalen RNA-Operon (Prrn), das zur Funktion in einem Pflanzenplastid geeignet ist;
(b) Ribosomenbindungsstelle des T7-Bakteriophagen-Polymerase-Gen-10-Leaders, der mit der Promotorkomponente (a) verknüpft ist;
(c) eine DNA-Sequenz, die die Agrobacterium-CP4-5-Enolpyruvylshikimat-3-phosphat-Synthase (EPSPS) codiert, welche einer Pflanzenzelle Toleranz gegenüber wenigstens der Herbizidverbindung Glyphosat verleihen kann, wenn die DNA-Sequenz in Plastiden der Pflanzenzelle transcribiert wird; und
(d) einen Transcriptionsterminationsbereich.

2. Konstrukt gemäß Anspruch 1, wobei das Konstrukt weiterhin Folgendes umfasst: (e) ein Gen, das einen Selektionsmarker für die Selektion von Pflanzenzellen umfasst, die ein diesen Marker exprimierendes Plastid umfassen, und (f) DNA-Bereiche, die dem Genom dieses Plastids homolog sind, wobei die homologen Bereiche in (f) die Komponenten (a), (b), (c), (d) und (e) flankieren.

3. Konstrukt gemäß Anspruch 1 oder 2, das weiterhin eine Sequenz enthält, die die 14 N-terminalen Aminosäuren des GFP am N-Terminus des durch (c) codierten Proteins codiert.

4. Pflanzenzellenplastid, das das Konstrukt gemäß einem der Ansprüche 1 bis 3 enthält.

5. Pflanze, Pflanzensamen, Pflanzenzelle oder Nachkommen davon, die ein Pflanzenplastid gemäß Anspruch 4 enthalten.

6. Verfahren zur Erzeugung von Toleranz gegenüber einem Herbizid in einer Pflanzenzelle, wobei das Verfahren Folgendes umfasst: Transformieren von Plastiden der Pflanzenzelle mit einem Konstrukt, wie es in einem der Ansprüche 1 bis 3 definiert ist, und Wachsenlassen von Pflanzenzellen, die die transformierten Plastide umfassen, unter Bedingungen, bei denen die DNA-Sequenz transcribiert wird, wodurch Pflanzenzellen, die die Pflanzenplastiden enthalten, tolerant gegenüber Anwendungen von Glyphosat werden.

7. Verfahren gemäß Anspruch 6, wobei das Konstrukt weiterhin eine zweite interessierende DNA-Sequenz umfasst.

8. Verfahren gemäß Anspruch 7, wobei die zweite DNA-Sequenz ein Gen umfasst, das ausgehend von einem Promotor exprimiert wird, der von dem Promotor (a) unabhängig ist.

9. Verfahren gemäß Anspruch 7, wobei die zweite DNA-Sequenz ein Gen umfasst, das ausgehend von dem Promotor (a) als polycistronische mRNA zusammen mit der DNA-Sequenz in (c) exprimiert wird.

10. Verfahren gemäß Anspruch 7, wobei die zweite DNA-Sequenz ein Gen umfasst, das kein Gen ist, welches einer Pflanzenzelle Toleranz gegenüber der Herbizidverbindung verleihen kann.

11. Verfahren gemäß Anspruch 7, wobei die zweite DNA-Sequenz ein Gen umfasst, das einer Pflanzenzelle Toleranz gegenüber einer zweiten Herbizidverbindung verleihen kann.

12. Herbizidtolerante Pflanzenzelle, die nach dem Verfahren von Anspruch 6 erhältlich ist.

13. Pflanze, Pflanzensamen oder Pflanzenteil, die bzw. der eine Pflanzenzelle gemäß Anspruch 12 umfasst.

14. Pflanzenzelle gemäß Anspruch 12, die insgesamt mehr als etwa 0,01% lösliche Proteine als Protein, das ausgehend von dem Herbizidtoleranz-Gen exprimiert wird, umfasst.

15. Pflanzenzelle gemäß Anspruch 12, die insgesamt mehr als etwa 0,1%, vorzugsweise mehr als etwa 0,2%, lösliche Proteine als Protein, das ausgehend von dem Herbizidtoleranz-Gen exprimiert wird, umfasst.

16. Pflanzenzelle gemäß Anspruch 12, die ein Konstrukt gemäß Anspruch 3 aufweist und insgesamt 1% oder mehr, vorzugsweise 12% oder mehr, lösliche Proteine als Protein, das ausgehend von dem Herbizidtoleranz-Gen exprimiert wird, umfasst.

17. Pflanze, Pflanzensamen oder Pflanzenteil, die bzw. der eine Pflanzenzelle gemäß Anspruch 16 umfasst.

18. Verfahren, bei dem die durch das Verfahren von Anspruch 6 erhältliche Herbizidtoleranz verwendet wird, um Zellen zu selektieren, die aus untransformierten Zellen durch das Konstrukt transformiert wurden.

19. Verfahren gemäß Anspruch 18, wobei die Selektion anhand der Herbizidtoleranz auf einem Medium erfolgt, das Glyphosat in einer Konzentration von wenigstens etwa 50 µM bis etwa 200 µM enthält.

20. Verfahren gemäß Anspruch 19, wobei die Selektion anhand der Herbizidtoleranz auf einem Medium erfolgt, das Glyphosat in einer Konzentration ab etwa 1 mM enthält.

21. Pflanzenzelle, die nach dem Verfahren von Anspruch 18 erhältlich ist.

## Revendications

1. Construction comprenant les composants suivants dans le sens 5' vers 3' de la transcription :
(a) une séquence promotrice de l'opéron ARN ribosomique 16S (Prrn) fonctionnel dans un plaste végétal ;
(b) un site de liaison au ribosome du gène 10 de la polymérase du bactériophage T7 leader joint au dit composant promoteur (a) ;
(c) une séquence d'ADN codant la CP4 5-énolpyruvylshikimate-3-phosphate synthase (EPSPS) d'Agrobacterium qui est capable de conférer la tolérance dans une cellule végétale à au moins le composé herbicide glyphosate lorsque ladite séquence d'ADN est transcrite dans les plastes de ladite cellule végétale ; et
(d) une région de terminaison de la transcription.

2. Construction selon la revendication 1, dans laquelle ladite construction comprend en outre (e) un gène codant un marqueur pouvant être choisi pour la sélection de cellules végétales comprenant un plaste exprimant ledit marqueur et (f) des régions d'ADN en homologie avec le génome dudit plaste, dans laquelle lesdites régions en homologie dans (f) flanquent les composants (a), (b), (c), (d) et (e).

3. Construction selon la revendication 1 ou 2, qui contient en outre une séquence codant les 14 acides aminés N-terminaux provenant de la GFP à la terminaison N de la protéine codée par (c).

4. Plaste de cellule végétale contenant la construction selon l'une quelconque des revendications 1 à 3.

5. Plante, graine végétale, cellule végétale ou progéniture de celle-ci contenant un plaste végétal selon la revendication 4.

6. Procédé de production de la tolérance à un herbicide chez une cellule végétale dans lequel ledit procédé comprend la transformation des plastes de ladite cellule végétale avec une construction telle que définie dans l'une quelconque des revendications 1 à 3, et culture des cellules végétales comprenant lesdits plastes transformés sous des conditions dans lesquelles ladite séquence d'ADN est transcrite moyennant quoi les cellules végétales contenant lesdits plastes végétaux sont rendues tolérantes aux applications de glyphosate.

7. Procédé selon la revendication 6, dans lequel ladite construction comprend en outre une seconde séquence d'ADN d'intérêt.

8. Procédé selon la revendication 7, dans lequel ladite seconde séquence d'ADN comprend un gène exprimé à partir d'un promoteur indépendant dudit promoteur (a).

9. Procédé selon la revendication 7, dans lequel ladite seconde séquence d'ADN comprend un gène exprimé à partir dudit promoteur (a) comme message polycistronique avec ladite séquence d'ADN dans (c).

10. Procédé selon la revendication 7, dans lequel ladite seconde séquence d'ADN comprend un gène autre qu'un gène capable de conférer dans une cellule végétale la tolérance au dit composé herbicide.

11. Procédé selon la revendication 7, dans lequel ladite seconde séquence d'ADN comprend un gène capable de conférer dans une cellule végétale la tolérance à un second composé herbicide.

12. Cellule végétale tolérante à un herbicide pouvant être obtenue par le procédé selon la revendication 6.

13. Plante, graine végétale ou partie de plante comprenant une cellule végétale selon la revendication 12.

14. Cellule végétale selon la revendication 12, qui comprend plus d'environ 0,01 % de protéines solubles totales comme une protéine exprimée à partir dudit gène de tolérance à un herbicide.

15. Cellule végétale selon la revendication 12, qui comprend plus d'environ 0,1 %, de préférence plus d'environ 0,2 % de protéines solubles totales comme protéine exprimée à partir dudit gène de tolérance à un herbicide.

16. Cellule végétale selon la revendication 12, qui a une construction telle que définie dans la revendication 3 et qui comprend 1 % ou plus, de préférence 12 % ou plus de protéines solubles totales sous la forme d'une protéine exprimée à partir dudit gène de tolérance à un herbicide.

17. Plante, graine végétale ou partie de plante comprenant une cellule végétale selon la revendication 16.

18. Procédé dans lequel la tolérance à l'herbicide pouvant être obtenue par le procédé selon la revendication 6 est utilisé pour choisir des cellules transformées par ladite construction à partir de cellules non transformées.

19. Procédé selon la revendication 18, dans lequel la sélection pour ladite tolérance à un herbicide est réalisée sur des milieux contenant du glyphosate à une concentration allant d'au moins environ 50 µM jusqu'à environ 200 µM.

20. Procédé selon la revendication 19, dans lequel la sélection pour ladite tolérance à un herbicide est réalisée sur des milieux contenant du glyphosate à une concentration d'environ 1 mM.

21. Cellule végétale pouvant être obtenue par le procédé selon la revendication 18.
